**Europäisches Patentamt**

**European Patent Office**

**Office européen des brevets**

(19)

(11) Numéro de publication : **0 421 863 B1**

# (12) FASCICULE DE BREVET EUROPEEN

(45) Date de publication du fascicule du brevet :
**12.01.94 Bulletin 94/02**

(51) Int. Cl.⁵ : **A61K 9/28**

(21) Numéro de dépôt : **90402713.3**

(22) Date de dépôt : **01.10.90**

(54) **Eléments thérapeutiques pour l'administration orale d'un médicament aux animaux et procédé de fabrication.**

(30) Priorité : **02.10.89 FR 8912831**

(43) Date de publication de la demande :
**10.04.91 Bulletin 91/15**

(45) Mention de la délivrance du brevet :
**12.01.94 Bulletin 94/02**

(84) Etats contractants désignés :
**AT BE CH DE FR IT LI LU NL**

(56) Documents cités :
**EP-A- 0 088 051**
**EP-A- 0 208 528**
**EP-A- 0 240 826**
**WO-A-89/12393**
**FR-A- 2 032 164**
**US-A- 4 650 673**

(73) Titulaire : **RHONE MERIEUX**
**17, rue Bourgelat**
**F-69002 Lyon (FR)**

(72) Inventeur : **Languet, Bernard**
**2 Les Barlettes**
**F-38138 Les Cotes d'Arey (FR)**
Inventeur : **Desmettre, Philippe**
**15 rue Tabard**
**F-69130 Ecully (FR)**

(74) Mandataire : **Bernasconi, Jean et al**
**CABINET LEMOINE ET BERNASCONI 13,**
**Boulevard des Batignolles**
**F-75008 Paris (FR)**

## Description

L'invention a pour objet des éléments thérapeutiques pour l'administration orale d'un médicament tel que vaccin, substance médicamenteuse ou analogue à des animaux sauvages ou domestiques.

L'invention a aussi pour objet un procédé de fabrication desdits éléments thérapeutiques.

La prise de médicaments par les animaux domestiques est le plus souvent réalisée par voie parentérale après contention.

Lorsque ces animaux sont élevés de manière extensive, il est difficile et parfois dangereux de les contenir, afin de leur administrer vaccins ou médicaments.

Pour les animaux sauvages, la capture de quelques individus est envisageable avec les mêmes difficultés et les mêmes risques, mais il est irréaliste de vouloir traiter tous les individus d'une même espèce dans un territoire donné.

On a envisagé la mise au point d'éléments contenant des médicaments efficaces et rendus appétants en vue de leur administration orale et distribuables sur de larges territoires afin de traiter des effectifs importants d'animaux sauvages, mais éventuellement aussi des individus appartenant à des espèces d'animaux domestiques que l'on ne souhaite ou que l'on ne peut pas capturer.

D'après la demande de brevet européen EP-A-240 826, on connaît un procédé de fabrication d'un appât, dans lequel on coule une substance de support, comprenant un composé lipidique, un composé destiné à stabiliser la forme de l'appât et une matière attractive et appétante vis-à-vis des animaux, au fond d'un moule, on met en place la substance active sur la couche obtenue puis on coule à nouveau de la substance de support de façon à enfermer la substance active dans une gangue de substance de support.

Ce procédé présente un certain nombre d'inconvénients. La coulée nécessite l'utilisation de substances assez fluides aux températures de coulée et, si l'on veut rendre cette température compatible avec la conservation du principe actif, il faut recourir à des substances dont le point de fusion peu élevé va se situer au voisinage des températures extrêmes d'utilisation, avec tous les inconvénients que cela peut poser concernant la stabilité de l'appât. En outre, la coulée de substance en deux temps peut conduire à une fragilité non négligeable au voisinage du plan de joint, ce qui, joint à la faible résistance mécanique de la substance, peut rendre l'appât inapte aux modes de distribution à grande échelle actuels, qui font appel notamment au largage aérien.

La présente invention a donc pour objectif de fournir des éléments thérapeutiques, pour l'administration orale d'un médicament aux animaux, qui, à la fois, soient attractifs et appétants vis-à-vis de l'animal concerné et présentent une résistance mécanique et thermique élevée, permettant la distribution par largage aérien dans toutes les conditions climatiques de la zone de distribution.

Un autre objectif de l'invention est de fournir de tels éléments thérapeutiques dont la structure, la texture et les dimensions facilitent la prise effective du médicament par l'animal.

Un autre objectif encore de l'invention est de fournir de tels éléments qui soient peu coûteux et facilement réalisables.

Un autre objectif encore de l'invention est de réaliser de tels éléments qui présentent une grande résistance à l'altération dans le milieu où ils sont distribués, et ce sur une longue période de temps.

La présente invention a aussi pour objectif de fournir un procédé de fabrication de ces éléments.

Un autre objectif de l'invention est de fournir un tel procédé applicable même à des médicaments extrêmement sensibles à la chaleur.

Un autre objectif encore de l'invention est de fournir un procédé facile à mettre en oeuvre, peu onéreux et facilement automatisable.

L'invention a pour objet un élément thérapeutique pour l'administration orale d'un médicament aux animaux, comprenant un principe actif contenu dans une enveloppe appétante, caractérisé en ce que l'enveloppe comprend, en mélange intime, un ou plusieurs matériaux appétants et une ou plusieurs substances agglomérant le matériau appétant et possédant une résistance mécanique et thermique élevée, préalablement conformée selon une forme creuse appropriée à la consommation par l'animal, et délimitant un volume interne, et en ce que ledit volume interne est rempli par un matériau liant appétant contenant le principe actif et épousant la forme interne de l'enveloppe pour assurer une continuité entre l'enveloppe et ledit principe actif.

De préférence, l'enveloppe a une forme tubulaire, notamment parallélépipédique.

Avantageusement, l'enveloppe peut être hydrophobe, soit en utilisant des substances agglomérantes hydrophobes, soit en incorporant un additif hydrophobe tel qu'une huile, etc.

Le matériau liant appétant peut être similaire à l'enveloppe. Il possède de préférence une, plusieurs ou la totalité des propriétés suivantes :
- il est résistant à la chaleur ;
- il participe, en liaison intime avec l'enveloppe, à l'élasticité ou à la plasticité de l'élément afin d'en augmenter la résistance au choc ;

- il est hydrophobe ;
- il apparaît à la surface, notamment lorsque l'enveloppe est tubulaire.

Le médicament peut être contenu dans un contenant tel que notamment sachet, gélule, capsule, etc. ou encore sous forme d'un comprimé ou analogue.

Le médicament peut aussi être disséminé dans le matériau liant ou mélangé à celui-ci.

Le médicament peut être sous forme solide ou liquide.

La matière agglomérante peut être notamment choisie parmi: polyosides, amidons, cellulose, cires végétales ou animales, silicones, polymères synthétiques, notamment hydrophobes, acides gras à longues chaînes, de préférence en $C_{20}$ ou plus, etc.

La matière agglomérante peut être notamment un polymère du type de ceux utilisés dans la constitution d'aliments pour crevettes et poissons selon les brevets US n° 4.576.821, n° 4.666.717 et n° 4.741.904, par exemple des acétates d'éthylène-vinyle.

L'enveloppe selon l'invention peut notamment avoir l'une des compositions décrites dans ces brevets US ou encore la composition de l'aliment pour crevettes vendu sous la marque CRAWDEAUX par la société américaine DU PONT DE NEMOURS.

Pour les renards et les chiens, les appâts peuvent avoir, de préférence, environ 50 mm de long, 32 mm de large, 20 mm de hauteur et la cavité de l'enveloppe tubulaire peut avoir environ 50 mm de long, 23 mm de large, 9 mm de hauteur.

Selon un mode de réalisation particulier de l'invention, l'appât peut comprendre un marqueur non toxique, par exemple antibiotique tel que le chlorydrate de tétracycline. Celui-ci peut notamment être contenu dans l'enveloppe, notamment à raison de 150 mg par appât.

L'invention a également pour objet un procédé de fabrication de tels éléments thérapeutiques, caractérisé en ce qu'on met en forme une enveloppe comprenant, en mélange intime, un ou plusieurs matériaux appétants et une ou plusieurs substances agglomérant le matériau appétant et possédant une résistance mécanique et thermique élevée, la mise en forme conférant à l'enveloppe une forme creuse délimitant un volume interne et en ce qu'on met en place dans le volume interne le principe actif et le matériau liant appétant, de façon que le matériau liant épouse la forme interne de l'enveloppe.

De préférence, on donne à l'enveloppe une forme tubulaire, notamment parallélépipédique. Cette enveloppe peut aussi avoir toute autre forme appropriée, notamment cylindrique.

De préférence, on réalise l'enveloppe par extrusion.

Dans un premier mode de réalisation préféré de l'invention, on met en place le principe actif dans le volume interne de l'enveloppe, puis on coule dans celui-ci le matériau liant.

Dans un deuxième mode de réalisation préféré de l'invention, on coule, dans le volume interne de l'enveloppe, le matériau liant contenant le principe actif.

Le procédé selon l'invention pourra facilement être appliqué à l'échelle industrielle.

L'élément préparé selon ce procédé pourra être conservé à température ordinaire ou à basse température selon les exigences de conservation du principe actif.

L'invention va être maintenant décrite plus en détail à l'aide d'un exemple de procédé selon l'invention, illustré par le dessin annexé dans lequel :

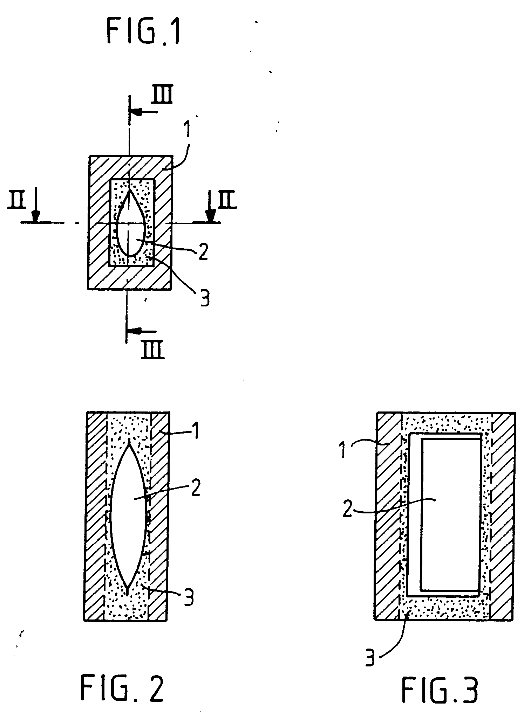

la figure 1 montre, en coupe transversale, un élément fabriqué à l'aide du procédé selon l'invention,

la figure 2 montre une vue en coupe longitudinale selon la ligne II-II de la figure 1,

la figure 3 montre une vue en coupe longitudinale selon la ligne III-III de la figure 1.

On a d'abord réalisé une enveloppe tubulaire 1 par un procédé d'extrusion aux environs de 100°C. Le mélange extrudé est formé au départ d'huile de poisson, d'aliments pour poisson et d'acétate d'éthylène-vinyle dans les proportions préférées des brevets US précités.

Les dimensions de l'enveloppe 1 sont choisies en fonction des animaux auxquels sont destinés les éléments thérapeutiques.

Une fois l'enveloppe 1 refroidie (l'enveloppe est relativement rigide et est hydrophobe) et mise à la longueur voulue, on place dans la cavité de l'enveloppe un sachet 2 en matière plastique (polyéthylène thermosoudé) contenant le principe actif (vaccin antirabique par exemple) sous un volume de 2,5 ml.

Ensuite, on coule dans la cavité une substance de liaison 3 composée d'un mélange de corps gras possédant un point de fusion peu élevé, restant pâteux aux températures élevées et n'étant pas cassant aux basses températures.

Ce mélange de corps gras comprend en outre une substance attractive et appétante vis-à-vis de l'animal concerné.

Propriétés physiques et de conservation.

Des essais de tenue à des températures comprises entre -20°C et +45°C ont montré que la structure de l'élément obtenu comme ci-dessus était conservée dans cet intervalle de température. A cette dernière température, le liant a une consistance pâteuse qui ne nuit pas à l'efficacité grâce à la tenue de l'enveloppe.

Les essais ont également montré une très bonne résistance aux chocs lors de largages à partir d'un hélicoptère, à une hauteur supérieure à 100 m sur une surface bétonnée, aucun élément ne s'étant détérioré.

On a également réalisé un certain nombre d'essais visant à évaluer l'efficacité de l'élément selon l'invention chez le renard et le chien. Les résultats obtenus ont démontré la bonne attractivité de l'appât vis-à-vis des animaux et la structure de l'appât s'est révélée particulièrement appropriée à la prise effective du principe actif (bonne délivrance du principe actif liquide dans la cavité buccale).

Propriétés attractives.

Un premier essai a été effectué sur 10 chiens répartis en boxes individuels. Chaque chien a reçu un élément selon l'invention (procédé ci-dessus) mélangé à sa ration de nourriture.

24 heures après la distribution, 100 % des éléments avaient été consommés et le marqueur coloré utilisé a permis de conclure que le contenu du sachet a été répandu dans la gueule de 9 chiens sur 10.

Un second essai a été réalisé dans les mêmes conditions sur 18 chiens et a conduit aux mêmes résultats.

En outre, des chiens qui ont reçu dans les jours suivants 1 ou 2 éléments selon l'invention les ont tous consommés dans un délai de 24 heures.

Efficacité.

L'efficacité a été contrôlée dans le cadre d'une vaccination anti-rabique de carnivores par voie orale, le vaccin utilisé étant une recombinant Vaccine/glycoprotéine G dont l'activité a par ailleurs été démontrée.

18 renards roux ont été répartis en 3 groupes (A, B, C) de 6 individus, chaque animal étant en cage individuelle.

Le groupe A a reçu 1 élément au jour 0 (J0).

Le groupe B a reçu 2 éléments (à J0 et à J1).

Le groupe C a reçu 3 éléments (J0, J1 et J2).

4 renards ont été en outre gardés comme témoins.

Pour chaque renard, on a suivi l'évolution du taux d'anticorps anti-rabiques.

28 jours après la vaccination, les renards ont été éprouvés par voie intra-musculaire (dose virulente de virus rabique).

Tous les témoins non vaccinés sont morts.

Les résultats sont donnés dans le tableau suivant :

| | taux de séroconversion | taux de protection |
|---|---|---|
| groupe A | 4 | 5 |
| groupe B | 5 | 6 |
| groupe C | 5 | 5 |

On a effectué un essai analogue sur 18 chiens n'ayant jamais reçu de vaccin anti-rabique. Le groupe A a reçu 1 élément à J0, le groupe B, 2 éléments à J0 et J3 et le groupe C, 3 éléments, à J0, J3 et J4.

Les taux de séroconversion sont les suivants :
- groupe A = 5,
- groupe B = 6,
- groupe C = 6.

Le procédé selon l'invention peut s'appliquer à la fabrication d'éléments selon l'invention destinés aussi bien aux animaux sauvages qu'aux animaux domestiques. Parmi eux, on peut notamment citer : renards, mouffettes, ratons-laveurs, chacals, chiens errants, chiens viverrins, mangoustes, chats harets (pour l'administration de vaccins contre la rage par exemple) ainsi que des espèces domestiques telles que les bovins, ovins, caprins, équins, porcins, chiens et chats.

Bien entendu, le principe actif pourra également être solide, poudreux ou pâteux. Il pourra être constitué par un vaccin, un antibiotique, une hormone, un antiparasitaire, etc.

## Revendications

1. Eléments thérapeutiques pour l'administration orale d'un médicament aux animaux, comprenant un principe actif contenu dans une enveloppe appétante, caractérisés en ce que l'enveloppe (1) comprend, en mélange intime, un ou plusieurs matériaux appétants et une ou plusieurs substances agglomérant le matériau appétant et possédant une résistance mécanique et thermique élevée, préalablement conformée selon une forme creuse appropriée à la consommation par l'animal et délimitant un volume interne, et en ce que ledit volume interne est rempli par un matériau liant appétant (3) contenant le principe actif (2) et épousant la forme interne de l'enveloppe (1) pour assurer une continuité entre l'enveloppe (1) et ledit principe actif (2).

2. Eléments thérapeutiques selon la revendication 1, caractérisés en ce que l'enveloppe a une forme tubulaire.

3. Eléments thérapeutiques selon la revendication 1 ou 2, caractérisés en ce que l'enveloppe a une forme parallélépipédique.

4. Eléments thérapeutiques selon l'une quelconque des revendications 1 à 3, caractérisés en ce que l'enveloppe est hydrophobe.

5. Eléments thérapeutiques selon la revendication 4, caractérisés en ce que la ou les substances agglomérantes confèrent à l'enveloppe son caractère hydrophobe.

6. Eléments thérapeutiques selon la revendication 4 ou 5, caractérisés en ce que l'enveloppe comprend en outre un additif conférant à l'enveloppe son caractère hydrophobe.

7. Eléments thérapeutiques selon l'une quelconque des revendications 1 à 6, caractérisés en ce que le matériau liant appétant a une composition analogue à celle de l'enveloppe.

8. Eléments thérapeutiques selon l'une quelconque des revendications 1 à 7, caractérisés en ce que le matériau liant est résistant à la chaleur.

9. Eléments thérapeutiques selon l'une quelconque des revendications 1 à 9, caractérisés en ce que l'enveloppe et le matériau liant sont élastiques.

10. Eléments thérapeutiques selon l'une quelconque des revendications 1 à 9, caractérisés en ce que l'enveloppe et le matériau liant sont plastiques.

11. Eléments thérapeutiques selon l'une quelconque des revendications 1 à 10, caractérisés en ce que le matériau liant est hydrophobe.

12. Eléments thérapeutiques selon la revendication 2 et l'une quelconque des revendications 1, 3 à 11, caractérisés en ce que le matériau liant apparaît à la surface de l'élément.

13. Elements thérapeutiques selon l'une quelconque des revendications 1 à 12, caractérisés en ce que le médicament est contenu dans un contenant.

14. Eléments thérapeutiques selon l'une quelconque des revendications 1 à 12, caractérisés en ce que le médicament est disséminé ou mélangé dans le matériau liant.

15. Procédé de fabrication d'éléments thérapeutiques tels que définis à la revendication 1, caractérisé en ce qu'on met en forme une enveloppe comprenant, en mélange intime, un ou plusieurs matériaux appétants et une ou plusieurs substances agglomérant le matériau appétant et possédant une résistance mécanique et thermique élevée, la mise en forme conférant à l'enveloppe une forme creuse appropriée à la consommation par l'animal et délimitant un volume interne, et en ce qu'on met en place dans ledit volume interne le principe actif et un matériau liant appétant, de façon que le matériau liant appétant épouse la forme interne de l'enveloppe.

16. Procédé selon la revendication 15, caractérisé en ce qu'on met en forme une enveloppe tubulaire.

17. Procédé selon la revendication 15 ou 16, caractérisé en ce qu'on met en forme l'enveloppe par un procédé d'extrusion.

18. Procédé selon l'une quelconque des revendications 15 à 17, caractérisé en ce que, dans le volume interne de l'enveloppe, on met en place le principe actif puis on assure le maintien du principe actif dans l'enveloppe en y coulant le matériau liant.

19. Procédé selon l'une quelconque des revendications 15 à 17, caractérisé en ce que, dans le volume interne de l'enveloppe, on coule le matériau liant contenant le principe actif.


**Patentansprüche**

1. Therapeutisches Element zur oralen Verabreichung eines Medikaments an Tiere bestehend aus einem aktiven Wirkstoff in einer die Freßlust anregenden Umhüllung (1) , dadurch gekennzeichnet, daß die Umhüllung, die zuvor entsprechend einer hohlen Form gebildet wurde, die für den Verzehr durch das Tier geeignet ist und die ein inneres Volumen begrenzt, in inniger Mischung ein oder mehrere die Freßlust anregende Materialien und ein oder mehrere Substanzen umfaßt, die das die Freßlust anregende Material agglomerieren und eine hohe mechanische und thermische Widerstandsfähigkeit besitzen, und dadurch, daß dieses innere Volumen mit einem die Freßlust anregenden Bindemittel (3) gefüllt wird, das den aktiven Wirkstoff (2) enthält und die innere Form der Umhüllung (1) annimmt, um eine Kontinuität zwischen der Umhüllung (1) und diesem aktiven Wirkstoff (2) sicherzustellen.

2. Therapeutische Elemente nach Anspruch 1, dadurch gekennzeichnet, daß die Umhüllung eine röhrenförmige Form hat.

3. Therapeutische Elemente nach Anspruch 1 oder 2, dadurch gekennzeichnet, daß die Umhüllung eine parallelflache Form hat.

4. Therapeutische Elemente nach irgendeinem der Ansprüche 1 bis 3, dadurch gekennzeichnet, daß die Umhüllung hydrophob ist.

5. Therapeutische Elemente nach Anspruch 4, dadurch gekennzeichnet, daß die agglomerierenden Substanzen der Umhüllung ihren hydrophoben Charakter vermitteln.

6. Therapeutische Elemente nach Anspruch 4 oder 5, dadurch gekenzeichnet, daB die Umhüllung auBerdem ein Additiv enthält, das der Umhüllung ihren hydrophoben Charakter vermittelt.

7. Therapeutische Elemente nach irgendeinem der Ansprüche 1 bis 6, dadurch gekennzeichnet, daß das die Freßlust anregende Bindemittel eine Zusammensetzung ähnlich derjenigen der Umhüllung hat.

8. Therapeutische Elemente nach irgendeinem der Ansprüche 1 bis 7, dadurch gekennzeichnet, daß das Bindemittel hitzebeständig ist.

9. Therapeutische Elemente nach irgendeinem der Ansprüche 1 bis 8, dadurch gekennzeichnet, daß die Umhüllung und das Bindemittel elastisch sind.

10. Therapeutische Elemente nach irgendeinem der Ansprüche 1 bis 9, dadurch gekennzeichnet, daß die Umhüllung und das Bindemittel plastisch sind.

11. Therapeutische Elemente nach irgendeinem der Ansprüche 1 bis 10, dadurch gekennzeichnet, daß das Bindemittel hydrophob ist.

**12.** Therapeutische Elemente nach Anspruch 2 und irgendeinem der Ansprüche 1 und 3 bis 11, dadurch gekennzeichnet, daß das Bindemittel an der Oberfläche des Elements erscheint.

**13.** Therapeutische Elemente nach irgendeinem der Ansprüche 1 bis 12, dadurch gekennzeichnet, daß das Medikament in einem Behältnis enthalten ist.

**14.** Therapeutische Elemente nach irgendeinem der Ansprüche 1 bis 12, dadurch gekennzeichnet, daß das Medikament im Bindemittel verteilt oder vermischt ist.

**15.** Verfahren zur Herstellung von wie in Anspruch 1 definierten therapeutischen Elementen, dadurch gekennzeichnet, daß man eine Umhüllung formt, die in inniger Mischung ein oder mehrere die Freßlust anregende Materialien und ein oder mehrere Substanzen umfaßt, die das die Freßlust anregende Material agglomerieren und eine hohe mechanische und thermische Widerstandsfähigkeit besitzen, wobei die Formgebung der Umhüllung eine hohle Form vermittelt, die für den Verzehr durch das Tier geeignet ist und die ein inneres Volumen begrenzt, und dadurch, daß man in dieses innere Volumen den aktiven Wirkstoff und das die Freßlust anregende Bindemittel so einbringt, daß das Bindemittel die innere Form der Umhüllung annimmt.

**16.** Verfahren nach Anspruch 15, dadurch gekennzeichnet, daß man eine tubuläre Umhüllung bildet.

**17.** Verfahren nach Anspruch 15 oder 16, dadurch gekennzeichnet, daß man die Umhüllung durch ein Extrusionsverfahren bildet.

**18.** Verfahren nach irgendeinem der Ansprüche 15 bis 17, dadurch gekennzeichnet, daß man in das innere Volumen der Umhüllung den aktiven Wirkstoff einbringt und dann den Fortbestand des aktiven Wirkstoffs in der Umhüllung sicherstellt, dadurch daB man das Bindemittel hineingieBt.

**19.** Verfahren nach irgendeinem der Ansprüche 15 bis 17, dadurch gekennzeichnet, daß man in des innere Volumen der Umhüllung das den aktiven Wirkstoff enthaltende Bindemittel gießt.


## Claims

**1.** Therapeutic elements for oral administration of medication to animals, comprising an active principle contained in a craved for envelope, characterized in that said envelope comprises, in admixture, one or more craved for materials and one or more substances agglomerating said materials and having a high thermal and mechanical resistance, said envelope being previously formed in a hollow shape appropriate for consumption by the animal and defining an internal volume, and characterized in that said internal volume is filled by a craved for binding material containing said active principle and closely conforming to the internal shape of said envelope in order to provide a continuity between said envelope and said active principle.

**2.** Therapeutic elements according to claim 1, characterized in that said envelope has a tubular shape.

**3.** Therapeutic elements according to claim 1 or 2, characterized in that said envelope has a parallelepiped shape.

**4.** Therapeutic elements according to one of claims 1 to 3, characterized in that said envelope is hydrophobic.

**5.** Therapeutic elements according to claim 4, characterized in that the agglomerating substances confer a hydrophobic character to said envelope.

**6.** Therapeutic elements according to claim 4 or 5, characterized in that said envelope further comprises an additive conferring a hydrophobic character to said envelope.

**7.** Therapeutic elements according to one of claims 1 to 6, characterized in that said craved for binding material has a composition analogous to that of said envelope.

**8.** Therapeutic elements according to one of claims 1 to 7, characterized in that said binding material is heat resistant.

9. Therapeutic elements according to one of claims 1 to 8, characterized in that said envelope and said binding material are elastic.

10. Therapeutic elements according to one of claims 1 to 9, characterized in that said envelope and said binding material are plastic.

11. Therapeutic elements according to one of claims 1 to 10, characterized in that said binding material is hydrophobic.

12. Therapeutic elements according to claim 2 and to one of claims 1, 3 to 11, characterized in that said binding material is apparent at the surface of said element.

13. Therapeutic elements according to one of claims 1 to 12, characterized in that said medication is contained in a container.

14. Therapeutic elements according to one of claims 1 to 12, characterized in that said medication is disseminated or admixed in the binding material.

15. Process for preparing therapeutic elements according to claim 1, characterized in that there is formed an envelope comprising, in admixture, one or more craved for materials and one or more substances agglomerating said craved for materials and possessing a high mechanical and thermal resistance, said shaping conferring to said envelope a hollow shape appropriate for animal consumption and defining an internal volume and characterized in that there is placed in said internal volume said active principle and a craved for binding material, in such a manner that said craved for binding material closely conforms to the internal shape of said envelope.

16. Process according to claim 15, characterized in that there is formed a tubular envelope.

17. Process according to claim 15 or 16, characterized in that said envelope is formed by an extrusion process.

18. Process according to one of claims 15 to 17, characterized in that said active principle is placed in the internal volume of said envelope and maintained in said envelope by casting said binding material in said internal volume.

19. Process according to one of claims 15 to 17, characterized in that said binding material containing said active principle is cast in said internal volume of said envelope.

## FIG.1

## FIG. 2

## FIG.3